# EUROPÄISCHE PATENTSCHRIFT

(11) **EP 0 723 953 B1**
(45) Veröffentlichungstag und Bekanntmachung des Hinweises auf die Patenterteilung: **19.07.2000**
(21) Anmeldenummer: 96100326.6
(22) Anmeldetag: 11.01.1996
(51) Int. Cl.: C07C 241/02, C07C 243/22

(54) **Verfahren zur Herstellung von 2-Fluorphenylhydrazin**
Process for the preparation of 2-fluorophenylhydrazine
Procédé de préparation de 2-fluorophényl hydrazine

(30) Priorität: 24.01.1995 DE 19501948
(43) Veröffentlichungstag der Anmeldung: 31.07.1996
(73) Patentinhaber: Clariant GmbH, 65929 Frankfurt am Main (DE)
(72) Erfinder: Arndt, Otto, Dr., D-65719 Hofheim (DE)

(56) Entgegenhaltungen:
- CH-A- 475 203
- DD-A- 220 841
- DE-B- 1 180 375
- PATENT ABSTRACTS OF JAPAN vol. 11, no. 80 (C-409) [2527] , 11.März 1987 & JP-A-61 233656 (NIPPON NOHYAKU CO LTD), 17.Oktober 1986,
- PATENT ABSTRACTS OF JAPAN vol. 5, no. 67 (C-53) [739] , 7.Mai 1981 & JP-A-56 016454 (MITSUBISHI KASEI KOGYO K.K.), 17.Februar 1981,

## Beschreibung

Die Erfindung betrifft ein Verfahren zur Herstellung von 2-Fluorphenylhydrazin.

2-Fluorphenylhydrazin ist ein wertvolles Zwischenprodukt zur Herstellung von Pflanzenschutzmitteln.

Die Herstellung von halogenierten Phenylhydrazinen ist in der Literatur beschrieben.

In Methoden der Org. Chemie (Houben-Weyl), 4. Aufl. Bd. X/2 (1967) S. 178 ff. wird für die Herstellung dieser Verbindungsklasse die Hydrazinolyse von Halogenverbindungen empfohlen. Dort wird aber auch die Herstellung von 2-Chlorphenylhydrazin-Hydrochlorid durch Reduktion mit Natriumsulfit beschrieben. In diesem Fall werden jedoch nur 50 % Ausbeute erhalten.

Daß die Herstellung von Halogenphenylhydrazinen durch Reduktion der entsprechenden Diazoniumsalze mit Hydrogensulfit nicht zu befriedigenden Resultaten führt, ist auch in mehreren anderen Literaturstellen belegt:
In Ber. 66, 727 (1933) ist die Herstellung von 4-Fluorphenylhydrazin durch Reduktion mit Natriumsulfit/Zinkstaub beschrieben, die Ausbeute liegt hier bei ca. 75 %.
In J. Chem. Soc. 1953, S. 3326 und J. Chem. Soc. 1945, 530 wird auf die schlechten Ergebnisse der Reduktion mit Sulfit verwiesen und die Reduktion mit Zinnchlorid in großem Salzsäureüberschuß empfohlen.
Diese Methode führt bei mäßigen Ausbeuten zu einer hohen Abwasserbelastung.

In Acta Chimika Hungary 7 (1955) 65-68 wird für die Herstellung von Fluorphenylhydrazinen die Reduktion der Diazoverbindungen mit SO₂ als beste Methode zur Herstellung dieser Verbindungen beschrieben. Diese Vorgehensweise hat mehrere gravierende Nachteile: Durch die Verwendung von Schwefelsäure statt Salzsäure bei der Diazotierung ist ein um ca. 80 % höherer Einsatz an Säure erforderlich. Die beschriebene Reduktionsstufe ist durch eine sehr schlechte Raumausbeute (10 l Wasser pro Mol Fluoranilin), große Mengen SO₂ im Abgas und hohem Energieaufwand gekennzeichnet.

Die beschriebenen Methoden haben zudem überwiegend den Nachteil, daß die Freisetzung der Hydrazinbase aus dem Hydrochlorid nicht im Reaktionsgemisch erfolgt, sondern daß erst das Hydrochlorid isoliert und anschließend das Hydrazin unter Verwendung von Natronlauge und eines zusätzlichen Lösungsmittels als Extraktionsmittel freigesetzt wird.

In JP-A-61-233 656 wird ein Verfahren zur Herstellung von 2-Fluor-5-nitrophenyl-hydrazin durch Diazotierung von 2-Fluor-5-nitroanilin und Reduktion mit Sulfit beschrieben. Die Ausbeute an Zielprodukt ist jedoch unbefriedigend.

Es bestand also ein Bedarf nach einem Verfahren, daß es ermöglicht 2-Fluorphenylhydrazin in hoher Ausbeute bei geringer Abwasserbelastung herzustellen.

Diese Aufgabe wird gelöst durch ein Verfahren zur Herstellung von 2-Fluorphenylhydrazin oder 2-Fluorphenylhydrazin-Hydrochlorid, dadurch gekennzeichnet, daß man 2-Fluoranilin mit salpetriger Säure in Gegenwart von 3 bis 3,5 Mol Salzsäure pro Mol 2-Fluoranilin diazotiert, das erhaltene Diazoniumsalz mit Bisulfitlauge bei einem pH-Wert von 4,5 bis 7,5 zum 2-Fluorphenylhydrazin-α,β-disulfonat reduziert, dieses mit Salzsäure zum 2-Fluorphenylhydrazin-Hydrochlorid verseift, zur Neutralisation Alkalilauge so zusetzt, daß die Temperatur bis auf ca. 60°C ansteigt, wobei von pH 3,5 bis pH 4,0 eine Temperatur von 45 bis 50°C erreicht wird, anschließend auf 15 bis 30°C abkühlt, den Niederschlag von 2-Fluorphenylhydrazin abfiltriert und dieses gegebenenfalls mit Salzsäure zu 2-Fluorphenylhydrazin-Hydrochlorid umsetzt.

Als Bisulfitlauge wird vorteilhaft ein Gemisch von Alkalihydrogensulfit Alkalihydroxid eingesetzt, wobei die Natrium- und Kaliumsalze besonders geeignet sind. Dieses Gemisch kann sowohl als wäßrige Lösung als auch als Feststoff eingesetzt werden. Es hat sich bewährt die Reduktion des Diazoniumsalzes bei einem pH-Wert von 4,5 bis 7,5, insbesondere 5,6 bis 6,5 mit NaHSO₃/NaOH und die Neutralisation des 2-Fluorphenylhydrazin-Hydrochlorids wie vorstehend beschreiben durchzuführen. Zur Isolation des Produkts kühlt man auf 15 bis 30°C ab. Das so erhaltene Produkt kann in der Schmelze im Vakuum entwässert werden.

Besonders vorteilhaft läßt sich das Verfahren durchführen, indem man 2-Fluoranilin mit salpetriger Säure in Gegenwart eines definierten Überschusses an Salzsäure diazotiert (insbesondere 3,2 Mol HCI pro Mol 2-Fluoranilin), anschließend das Diazoniumsalz mit Natriumhydrogensulfit/NaOH (2,2 Mol NaOH, 2,2 Mol NaHSO₃ pro Mol 2-Fluoranilin) in einem bestimmten pH-Bereich (pH = 5,6 bis 6,5) zum 2-Fluorphenylhydrazin-α,β-disulfonat reduziert, dann das Disulfonat in der Hitze und in Gegenwart von Salzsäure zum 2-Fluorphenylhydrazin-Hydrochlorid verseift. Zur Neutralisation des Reaktionsgemisches des 2-Fluorphenylhydrazin-Hydrochlorids läßt man konzentrierte Natronlauge zulaufen, wobei während des Zulaufs der konzentrierten Natronlauge zum vorgelegten Reaktionsgemisch die Temperatur durch die Neutralisationswärme in dem Maße bis auf schließlich ca. 60°C ansteigt, so daß noch vor Beginn der Fällung (ab pH 3,5 bis 4,0) der ersten Anteile der freien Hydrazin-Base im Verlauf des Temperaturgradienten eine Temperatur erreicht wird, die oberhalb des Schmelzpunktes der freien Hydrazin-Base von ca. 45 bis 50°C in Gegenwart des Reaktionsgemischs liegt. Folglich ist der Anteil an gebildetem Feststoff der freien Hydrazin-Base im Reaktionsgemisch gering. Schließlich liegt ein flüssiges Zweiphasengemisch aus der wäßrigen Mutterlauge und einer ölartigen Phase der freien Hydrazin-Base vor.
Zur Isolierung der freien Hydrazin-Base hat es sich als günstig erwiesen, anschließend nach Erreichen des pH 8 bis 9 bei 50 bis 60°C auf 18 bis 22°C abzukühlen. Das geschieht vorteilhafterweise durch langsames Kühlen mit einer milden Außenkühlung, um die bei evtl. Unterkühlung veranlaßte Kristallisation von Natriumsulfat-deka-hydrat zu vermeiden.

Überraschenderweise erwies sich das ab ca. 40°C auskristallisierende Produkt als grobkörnig und granulatartig. Daraus folgt ein hoher Trockengehalt und geringer Gehalt an anorganischen Salzen. Es entfällt die Notwendigkeit einer Phasentrennung oberhalb des Schmelzpunktes zwecks Abtrennen der ausgeschiedenen Salzlauge.

Interessant am erfindungsgemäßen Verfahren ist, daß die Neutralisation des 2-Fluorphenylhydrazin-Hydrochlorids zum freiem 2-Fluorphenylhydrazin auch bei erhöhter Temperatur durchgeführt werden kann. Gegenüber dieser Vorgehensweise bestand ein Vorurteil in der Literatur, da z.B. nach Methoden der Organischen Chemie, Houben-Weyl, Bd. X/2 (1967), S. 178 bekannt war, daß Gemische aus Phenylhydrazin und Phenylhydrazin-Hydrochlorid schon unterhalb 100°C thermische Zersetzung zeigen.

Überraschenderweise konnte 2-Fluorphenylhydrazin durch Diazotierung und Bisulfit-Reduktion hergestellt werden, obwohl ein Vorurteil gegen diese Herstellungsmethode bestand (J. Chem. Soc. 1953, 3326 und J. Chem. Soc. 1945, 530).

Die erfindungsgemäße Verfahrensweise eröffnet eine vorteilhafte Möglichkeit, zur Herstellung des 2-Fluorphenylhydrazin-Hydrochlorids:
Die freie Hydrazin-Base ist schlecht zu handhaben, da das Befüllen und Entleeren der Behälter nur über die Schmelze erfolgen kann.
Das in Pulverform anfallende Hydrochlorid ist leichter zu handhaben. Das Hydrochlorid ist leichter zu trocknen, während sich das Restwasser aus der freien Hydrazin-Base nur durch technischen Aufwand entfernen läßt.
Es ist daher naheliegend, das Herstellverfahren ausgehend von 2-Fluoranilin so zu modifizieren, daß man das zwischendurch gebildete Hydrochlorid durch Filtration isoliert. Jedoch zeigte sich, daß diese Arbeitsweise ausgehend vom 2-Fluoranilin mit Nachteilen verbunden ist. Wegen der gegenüber der freien Hydrazin-Base erhöhten Löslichkeit des Hydrochlorids im Reaktionsgemisch ist ein erhöhter Einsatz an Salzsäure bei der Fällung nötig. Selbst dann resultiert noch ein deutlicher Ausbeuteverlust. Dies führt zu verstärkter Abwasserbelastung an organischem und anorganischem Material. Das isolierte kristalline Hydrochlorid enthält außerdem 5 bis 10 % Natriumsulfat.

Der genannte Ausbeuteverlust läßt sich ausgleichen, wenn man die salzsaure Mutterlauge des Hydrochlorids mit Natronlauge neutralisiert und mit Xylol extrahiert. Daraus erhält man 2-Fluorphenylhydrazin in einer Menge von ca. einem Drittel der Ausbeute. Diese große Menge muß dem nächsten Ansatz zugeführt werden. Diese Maßnahme ist aufwendig.

Überraschenderweise läßt sich nun aus der freien Hydrazin-Base, die nach dem oben geschilderten Verfahrensweg erhalten worden ist, das Hydrochlorid in hoher Ausbeute und gegenüber der Rohbase verbesserter Qualität herstellen. Voraussetzung hierfür ist, daß die Rohbase frei von Natriumsulfat ist. Dies ist gegeben, wenn man die oben geschilderten erfindungsgemäßen Bedingungen einhält. Hierzu wird die Rohbase mit Salzsäure umgesetzt, die Fällung des Hydrochlorids mit Salzsäure vervollständigt und das 2-Fluorphenylhydrazin-Hydrochlorid abfiltriert.

Die nachstehenden Beispiele dienen der Erläuterung des erfindungsgemäßen Verfahrens, ohne es zu beschränken. Teile sind hierbei Gewichtsteile:

### Beispiele

### Beispiel 1:

### Während des Versuchsablaufs wird ständig gerührt und mit Stickstoff überlagert.

### a) Herstellung der Rohbase

In ein Gemisch aus 400 Teilen Wasser, 310 Teilen Salzsäure 37 % werden 113,5 Teile 2-Fluoranilin eingetragen. Man läßt bei 20°C 174 Teile wäßrige Natriumnitrit-Lösung 40 % zulaufen. Nach Zerstören des Nitritüberschusses mit Amidoschwefelsäure läuft die Diazoniumsalzlösung bei 10 bis 15°C in ein Gemisch von 550 Teilen Natriumhydrogensulfit ca. 40 % und 108 Teilen Natriumhydroxid 50 % ein. Gleichzeitig mit dem weiteren Zulauf der Diazoniumsalzlösung werden ca. 70 Teile Natronlauge 50 % zugetropft, um den pH auf 5,4 bis 6,0 zu halten. Man heizt auf 70°C. Zu der noch 70°C heißen Lösung laufen unter Heizen auf 75 bis 80°C 245 Teile Salzsäure 37 %. Man rührt 30 Minuten nach und kühlt den Ansatz auf 30°C ab, wobei ab ca. 55°C das Hydrochlorid des 2-Fluorphenylhydrazins auskristallisiert. Man fügt einen aus der Mutterlauge der Rohbase und dem Waschfiltrat erhaltenen Recyclinganteil von ca. 7 Teilen des vorangegangenen Ansatzes hinzu. Man läßt ca. 360 Teile Natronlauge 50 % zulaufen und steigert gleichzeitig die Temperatur rampenförmig (linearer Gradient) von 30 auf ca. 60°C. Ab pH 3,8 bei 45°C beginnt zunächst die Fällung der freien Hydrazin-Base als Feststoff, der sich jedoch nicht anreichert, da nun bald das Schmelzen einsetzt. Die Neutralisation wird beendet bei pH 8,5 und ca. 60°C.

Der größte Teil des Produkts ist in der wäßrigen Phase gelöst. Das flüssige Zweiphasengemisch (2400 bis 2450 Teile) wird von 50 auf 20°C abgekühlt. Ab ca. 40 bis 45°C kristallisiert das 2-Fluorphenylhydrazin in gröberen Kristallen (Granulat) aus. Man filtriert und wäscht das Nutschgut mit 200 Teilen Wasser. Die Filtration dauert nur ca. 5 Minuten. Man erhält ca. 130 bis 180 Teile Rohbase mit einem Trockengehalt von 115 Teilen 2-Fluorphenylhydrazin, entspr. 91 % d. Th. Die Raumausbeute beträgt ca. 60 Teile pro 1000 Volumenteilen.

### b) Herstellung des Hydrochlorids

Zur Herstellung des Hydrochlorids wird die Rohbase bei 55 bis 60°C in einem beheizbaren Dosiertrichter aufgeschmolzen. Enthält sie noch Natriumsulfat, scheidet sich dieses als untere Phase aus und muß abgetrennt werden. Man läßt die Schmelze der Rohbase bei 60 bis 65°C in 164 Teile Salzsäure 20 % einlaufen. Dazu laufen anschließend langsam 90 Teile Salzsäure 37 % bei 60 bis 65°C. Man kühlt langsam auf 10°C ab. Man filtriert bei 10°C. Man erhält 145 Teile 2-Fluorphenylhydrazin-Hydrochlorid (89 % d.Th.) mit Schmelzpunkt 208 bis 210°C, wenn der oben erwähnte Recyclinganteil zugesetzt wurde. Der Gehalt an 2-Fluorphenylhydrazin-Hydrochlorid beträgt 99,0 bis 99,8 % (alkalimetrische und argentometrische Titration). Das Produkt enthält keine anorganische Salze.

## Patentansprüche

1. Verfahren zur Herstellung von 2-Fluorphenylhydrazin oder 2-Fluorphenylhydrazin-Hydrochlorid, dadurch gekennzeichnet, daß man 2-Fluoranilin mit salpetriger Säure in Gegenwart von 3 bis 3,5 Mol Salzsäure pro Mol 2-Fluoranilin diazotiert, das erhaltene Diazoniumsalz mit Bisulfitlauge bei einem pH-Wert von 4,5 bis 7,5 zum 2-Fluorphenylhydrazin-α,β-disulfonat reduziert, dieses mit Salzsäure zum 2-Fluorphenylhydrazin-Hydrochlorid verseift, zur Neutralisation Alkalilauge so zusetzt, daß die Temperatur bis auf ca. 60°C ansteigt, wobei von pH 3,5 bis pH 4,0 eine Temperatur von 45 bis 50°C erreicht wird, anschließend auf 15 bis 30°C abkühlt, den Niederschlag von 2-Fluorphenylhydrazin abfiltriert und dieses gegebenenfalls mit Salzsäure zu 2-Fluorphenylhydrazin-Hydrochlorid umsetzt.

2. Verfahren nach Anspruch 1, dadurch gekennzeichnet, daß als Bisulfitlauge ein Gemisch aus Alkalihydrogensulfit/Alkalihydroxid, insbesondere Natrium- oder Kaliumhydrogensulfit und -hydroxid, bevorzugt Natriumhydrogensulfit und Natriumhydroxid eingesetzt wird.

3. Verfahren nach Anspruch 1 oder 2, dadurch gekennzeichnet, daß die Reduktion des Diazoniumsalzes zum 2-Fluorphenylhydrazin-α,β-disulfonat bei einem pH-Wert von 5,6 bis 6,5 durchgeführt wird.

4. Verfahren nach mindestens einem der Ansprüche 1 bis 3, dadurch gekennzeichnet, daß als Alkalilauge NaOH verwendet wird.

5. Verfahren nach mindestens einem der Ansprüche 1 bis 4, dadurch gekennzeichnet, daß die Diazotierung des 2-Fluoranilins mit salpetriger Säure in Gegenwart von 3,2 Mol Salzsäure pro Mol 2-Fluoranilin durchgeführt wird.

6. Verfahren nach mindestens einem der Ansprüche 1 bis 5, dadurch gekennzeichnet, daß das Diazoniumsalz mit 2,2 Mol Natriumhydrogensulfit und 2,2 Mol NaOH pro Mol 2-Fluoranilin reduziert wird.

7. Verfahren nach mindestens einem der Ansprüche 1 bis 6, dadurch gekennzeichnet, daß bei Erreichen eines pH-Wertes von 8 bis 9 auf 18 bis 22°C abgekühlt wird.

8. Verfahren nach mindestens einem der Ansprüche 1 bis 7, dadurch gekennzeichnet, daß die Freisetzung der freien Base aus dem Hydrochlorid im Reaktionsgemisch erfolgt.

9. Verfahren nach mindestens einem der Ansprüche 1 bis 8, dadurch gekennzeichnet, daß die Abtrennung von Natriumsulfat aus der Rohbase durch Phasentrennung in der Produktschmelze bei 55 bis 60°C erfolgt.

10. Verfahren nach mindestens einem der Ansprüche 1 bis 9, dadurch gekennzeichnet, daß man die freie Base mit Salzsäure umsetzt, die Fällung des 2-Fluorphenylhydrazin-Hydrochlorids mit Salzsäure vervollständigt und das 2-Fluorphenylhydrazin-Hydrochlorid abfiltriert.

## Claims

1. A process for the preparation of 2-fluorophenylhydrazine or 2-fluorophenylhydrazine hydrochloride, which comprises diazotizing 2-fluoroaniline nitrous acid in the presence of 3 to 3.5 mol of hydrochloric acid per mole of 2-fluoroaniline, reducing the resulting diazonium salt with alkaline bisulfite solution at a pH of 4.5 to 7.5 to give 2-fluorophenylhydrazine α,β-disulfonate, hydrolyzing the latter with hydrochloric acid to give 2-fluorophenylhydrazine hydrochloride, adding alkali metal hydroxide solution to neutralize the mixture such that the temperature rises to about 60°C, a temperature of 45 to 50°C being reached from pH 3.5 to pH 4.0, subsequently cooling the mixture to 15 to 30°C, removing the 2-fluorophenylhydrazine precipitate by filtration and, if appropriate, reacting the latter with hydrochloric acid to give 2-fluorophenylhydrazine hydrochloride.

2. The process as claimed in claim 1, wherein the alkaline bisulfite solution employed is a mixture of alkali metal hydrogen sulfite/alkali metal hydroxide, in particular sodium hydrogen sulfite or potassium hydrogen sulfite and sodium hydroxide or potassium hydroxide, preferably sodium hydrogen sulfite and sodium hydroxide.

3. The process as claimed in claim 1 or 2, wherein the diazonium salt is reduced to the 2-fluorophenylhydrazine α,β-disulfonate at a pH of 5.6 to 6.5.

4. The process as claimed in at least one of claims 1 to 3, wherein NaOH is used as the alkali metal hydroxide solution.

5. The process as claimed in at least one of claims 1 to 4, wherein the 2-fluoroaniline is diazotized using nitrous acid in the presence of 3.2 mol of hydrochloric acid per mole of 2-fluoroaniline.

6. The process as claimed in at least one of claims 1 to 5, wherein the diazonium salt is reduced using 2.2 mol of sodium hydrogen sulfite and 2.2 mol of NaOH per mole of 2-fluoroaniline.

7. The process as claimed in at least one of claims 1 to 6, wherein the mixture is cooled to 18 to 22°C when a pH of 8 to 9 is reached.

8. The process as claimed in at least one of claims 1 to 7, wherein the free base is liberated from the hydrochloride in the reaction mixture.

9. The process as claimed in at least one of claims 1 to 8, wherein sodium sulfate is removed from the crude base by means of phase separation in the product melt at 55 to 60°C.

10. The process as claimed in at least one of claims 1 to 9, wherein the free base is reacted with hydrochloric acid, precipitation of the 2-fluorophenylhydrazine hydrochloride with hydrochloric acid is completed and the 2-fluorophenylhydrazine hydrochloride is filtered off.

## Revendications

1. Procédé pour la préparation de la 2-fluorophénylhydrazine ou du chlorhydrate de 2-fluorophénylhydrazine, caractérisé en ce que l'on diazote la 2-fluoroaniline au moyen d'acide nitreux en présence de 3 à 3,5 mol d'acide chlorhydrique par mol de 2-fluoroaniline, on réduit le sel de diazonium obtenu avec de la lessive de bisulfite à un pH de 4,5 à 7,5 pour obtenir de l'α,β-disulfonate de 2-fluorophénylhydrazine, on saponifie celui-ci avec de l'acide chlorhydrique pour obtenir le chlorhydrate de 2-fluorophénylhydrazine, pour neutraliser on ajoute de la lessive alcaline de sorte que la température augmente jusqu'à environ 60°C, une température de 45 à 50°C étant atteinte de pH 3,5 à pH 4,0, ensuite on refroidit à une température de 15 à 30°C, on sépare le précipité de 2-fluorophénylhydrazine par filtration et on transforme éventuellement celle-ci en chlorhydrate de 2-fluorophénylhydrazine au moyen d'acide chlorhydrique.

2. Procédé selon la revendication 1, caractérisé en ce qu'on utilise, en tant que lessive de bisulfite, un mélange de bisulfite alcalin/hydroxyde alcalin, en particulier du bisulfite et de l'hydroxyde de sodium ou de potassium, de préférence du bisulfite de sodium et de l'hydroxyde de sodium.

3. Procédé selon la revendication 1 ou 2, caractérisé en ce que la réduction du sel de diazonium en α,β-disulfonate de 2-fluorophénylhydrazine est effectuée à un pH de 5,6 à 6,5.

4. Procédé selon au moins une des revendications 1 à 3, caractérisé en ce que l'on utilise NaOH comme lessive alcaline.

5. Procédé selon au moins une des revendications 1 à 4, caractérisé en ce que la diazotation de la 2-fluoroaniline est effectuée au moyen d'acide nitreux en présence de 3,2 mol d'acide chlorhydrique par mol de 2-fluoroaniline.

6. Procédé selon au moins une des revendications 1 à 5, caractérisé en ce que le sel de diazonium est réduit au moyen de 2,2 mol de bisulfite de sodium et 2,2 mol de NaOH par mol de 2-fluoroaniline.

7. Procédé selon au moins une des revendications 1 à 6, caractérisé en ce que l'on refroidit à une température de 18 à 22°C lorsque l'on atteint un pH de 8 à 9.

8. Procédé selon au moins une des revendications 1 à 7, caractérisé en ce que la libération de la base libre à partir du chlorhydrate a lieu dans le mélange réactionnel.

9. Procédé selon au moins une des revendications 1 à 8, caractérisé en ce que la séparation du sulfate de sodium à partir de la base brute est effectuée par séparation de phase dans la masse fondue de produit à une température de 55 à 60°C.

10. Procédé selon au moins une des revendications 1 à 9, caractérisé en ce que l'on fait réagir la base libre avec de l'acide chlorhydrique, on complète la précipitation du chlorhydrate de 2-fluorophénylhydrazine au moyen d'acide chlorhydrique et on sépare le chlorhydrate de 2-fluorophénylhydrazine par filtration.
